# EUROPEAN PATENT SPECIFICATION

(11) **EP 1 599 731 B1**
(45) Date of publication and mention of the grant of the patent: **08.08.2007**
(21) Application number: 04716274.8
(22) Date of filing: 02.03.2004
(51) Int. Cl.: G01N 33/58, G01N 33/543

(54) **USE OF A VIRUS EXPRESSING A BINDING MOIETY TO MEASURE ANALYTES IN A SAMPLE**
VERWENDUNG EINES VIRUSES, DER EIN BINDUNGSTEIL EXPRIMIERT, ZUR MESSUNG VON ANALYTEN IN EINER PROBE
UTILISATION D'UN VIRUS EXPRIMANT UN FRAGMENT DE LIAISON POUR MESURER DES ANALYTES DANS UN ECHANTILLON

(30) Priority: 04.03.2003 GB 0304832
(43) Date of publication of application: 30.11.2005
(60) Divisional application: 07011811.2
(73) Proprietor: THE SECRETARY OF STATE FOR DEFENCE, Salisbury Wiltshire SP4 0JQ (GB)
(72) Inventor: SQUIRRELL, David James, Salisbury, Wiltshire SP4 0JQ (GB); LEE, Martin, Alan, Salisbury, Wiltshire SP4 0JQ (GB); MAYERS, Carl, Nicholas, Salisbury, Wiltshire SP4 0JQ (GB)
(74) Representative: Greaves, Carol Pauline
(86) International application number: PCT/GB2004/000865
(87) International publication number: WO 2004/079369

(56) References cited:
- EP-A- 0 402 997
- EP-A- 0 574 345
- US-A- 5 403 484
- US-A- 5 922 537
- US-A1- 2002 110 806
- US-B1- 6 190 856
- US-B1- 6 265 169
- US-B1- 6 524 809
- GOLDMAN ELLEN R ET AL: "Phage-displayed peptides as biosensor reagents" JOURNAL OF MOLECULAR RECOGNITION, vol. 13, no. 6, November 2000 (2000-11), pages 382-387, XP002285448 ISSN: 0952-3499
- PETRENKO VALERY A ET AL: "Phages from landscape libraries as substitute antibodies" PROTEIN ENGINEERING, vol. 13, no. 8, August 2000 (2000-08), pages 589-592, XP002285449 ISSN: 0269-2139
- KLEIN ET AL.: TRENDS IN MOLECULAR MEDICINE, vol. 8, no. 6, 8 May 2002 (2002-05-08), pages 257-260,
- EDWARDS MC AND GIBBS RA: PCR METHODS AND APPLICATIONS, COLD SPRING HARBOR LABORATORY PRESS, US, vol. 3, no. 4, February 1994 (1994-02), pages S65-S75,
- EKINS R AND CHU F: JOURNAL OF THE INTERNATIONAL FEDERATION OF CLINICAL CHEMISTRY, vol. 9, no. 3, September 1997 (1997-09), pages 100-109,

## Description

The present invention provides a novel assay method as well as kits useful in said assay.

Immuno-PCR, which dates from 1992, uses nucleic acid tagged antibodies to provide a very sensitive assay endpoint.
Generally the antibody is labelled with streptavidin and the nucleic acid through the streptavidin to the antibody. The biotinylated DNA is usually added at the end of the immunoassay.

Viruses may comprise essentially DNA or RNA, and attack host cells, integrating their nucleic acids into the host system. In structural terms, viruses generally express proteins which form a "coat" around the nucleic acids.

Phage display is a technique that was developed to allow proteins such as antibodies to be selected and produced *in vitro.* Phage libraries are made that contain a very high number of different proteins, such as scFv's (single chain variable fragments from antibodies). The phage has the DNA for the protein scFv's inserted into its genome and it expresses it as a fusion protein to the coat proteins, generally attached at its head.

The best protein such as scFv for a particular purpose is selected from the library mixture by "panning" for the phage that binds to the analyte of interest under strict selection conditions. The phage can then be multiplied by growth in its host bacterium and the DNA can be cut out and inserted into an expression vector. Binding protein can then be produced either as scFv or incorporated back into an antibody framework to make, for example, humanised antibodies for therapeutic use.

The phage display technique is thus used as an intermediate technique for *in vitro* antibody production. However, the phages themselves have never been proposed for use as assay reagents in a competitive immunoassay using quantitative PCR. US 6265169 discloses their use in a sandwich immunoassay using normal PCR.

The applicants have developed a particular method as defined in the claims in which virus, which expresses and displays a binding moiety is used as a detectable moiety in an assay for detecting the presence or measuring the concentration of an analyte in a sample.

As used herein, the expression "detectable moiety" means that the virus itself is detected to provide a signal indicative of the presence or absence of an analyte.

In addition, the expression "binding moiety" relates to any moiety which will bind to a target, especially a polypeptide or protein, which may be for example an analyte polypeptide or protein, but may also be another polypeptide or protein, which is utilised in an immunoassay as part of the detection system.

The nucleic acid of the virus acts as a label, which may be detected using any of the known nucleic acid detection methods, in particular by using amplification reactions such as the polymerase chain reaction. However, the advantage of using a virus as compared to any other labelling technique is that a wide variety of binding moieties can be included relatively simply using techniques known for example for the production of recombinant viruses, such as phage display libraries, and without the need for additional binding steps.

Any type of virus may be used in the context of the invention as described below, so that it may be a DNA virus, and in particular a bacteriophage (phage), which is a virus which attacks bacterial cells, but other DNA viruses or RNA viruses may also be employed. The virus is transformed so as to express and display a binding moiety as described in the claims. Thus, generally the virus will le a phage, and in particular a recombinant phage.

In particular, the virus will comprise a recombinant phage, which has been transformed so that expresses and displays a specific binding moiety such as an immunoglobulin for instance, an antibody, or a binding fragment thereof. However, the virus may be transformed to express any protein which may be of use in an immunoassay, including target analytes or analogues of these, even where these are not of the immunoglobulin superfamily.

Assay formats, which may use these reagents, may be any of the conventional competitive type assays.

Thus, the invention provides a method for determining the amount of an analyte in a sample, said method comprising contacting said sample with a surface having immobilised thereon a binding reagent which either (a) binds said analyte, or (b) comprises said analyte or an analogue thereof, and a virus which has been transformed so that it expresses and displays a binding moiety that binds said binding reagent in competition to said analyte, separating said surface from the sample, and carrying cut a quantitative polymerase chain reaction to detect the amount of a nucleic acid sequence present within said virus on said surface, wherein at least one of the binding reagent or the binding moiety is specific for the analyte.

Suitably, the virus is incubated in the presence of the surface for a sufficient period of time to ensure that it binds to available binding sites on the surface, for example to any binding reagent which is not occupied by analyte from the sample. For example, the incubation may take place for a period of from 5 to 60 minutes, at appropriate temperatures, such as from 25-40°C, for instance at about 37°C.

After this incubation, the surface including any immobilised complex is separated from the virus suspension, for example by removing the virus suspension and washing the surface.

Thereafter, a nucleic acid sequence, and in particular a nucleic acid, which may be a DNA or RNA, which is characteristic of the virus is detected on the surface using a quantitative polymerase chain reaction (PCR).

In a typical competitive type assay, a binding reagent for an analyte, or the analyte or an analogue thereof, is immobilised on a surface.

As used herein, the expression "analogue" refers to a moiety that will bind to a binding partner which binds the analyte, even though it may not be of precisely the same sequence or structure as the analyte. It may, for instance, comprise a particular epitopic region of an analyte, which is bound by a specific monoclonal antibody, which therefore acts as the binding partner. Thus an analogue will "mimic" an analyte in the context of an immunoassay using a common binding partner.

In one embodiment, a sample, to which a virus that expresses and displays a binding moiety for the analyte or analogue is added, is contacted with the surface. When analyte is present in the sample, it will compete with the immobilised analyte or analogue for binding to the virus. Thus, less virus will be retained upon the surface, than would be the case if no analyte was present in the sample. This reduction in the amount of retained virus can be detected in accordance with the invention, by analysing the surface for the presence of a nucleic acid present in the virus.

In an alternative competitive type assay, a binding reagent for the analyte is immobilised on the surface. In this case, the binding moiety displayed on the virus is a specific binding partner which is selected so that it competes with analyte for binding to the immobilised binding reagent. The less virus DNA detected on the surface after separation from the sample, the more analyte is present. Particular examples of analytes in this case are immunoglobulins such as antibodies, which may be useful in diagnosis of disease.

In all cases however, nucleic acid of the virus acts as detectable and specific "label" for the binding moiety, and may be detected using a polymerase chain reaction or PCR reaction, which may be specific for the particular virus nucleic acid. Quantification of the analyte in the sample is carried out using quantitative PCR methods, as are well known in the art.

In one embodiment, the immobilised binding reagent is an analogue of the analyte, which mimics the analyte in the sense that it will bind to the binding moiety of the virus in competition with the analyte. Thus the binding moiety will bind either the analyte or the binding reagent but not both. In this case, the sample is preferably incubated with the virus prior to contact with the surface. During this step, any analyte present will form a complex with the binding moiety on the virus, blocking the binding of the virus to the immobilised binding reagent on the surface. As a result, the complex will not be retained on the surface after washing and so the amount of detectable virus nucleic acid is reduced. In the absence of analyte, the binding moiety of the virus will be free to bind the binding reagent, resulting in a large viral nucleic acid "signal" being retained on the surface.

In some cases, the immobilised binding reagent binds the analyte, and also the binding moiety on the virus. In such cases, where the analyte is present in the sample, both the analyte and the binding moiety will compete for available sites on the surface. As a result, the amount of virus/binding moiety that is immobilised on the surface is reduced by an amount which relates to the concentration of analyte in the sample. Again, in this case, the presence of only a lower than expected "signal" from the viral nucleic acid is indicative of the presence of analyte in the sample.

This assay can be extremely sensitive, and background signals, which are associated with conventional immunoassay methods, can be reduced. The analysis itself is more readily controlled, as the virus can be engineered to comprise whatever sequence is convenient. The detection is entirely independent upon the nature of the analyte.

Analytes are generally proteins or polypeptides. Typical examples will be polypeptides or proteins that are associated with or part of a pathogenic organism such as a virus, bacteria or bacterial spore such as anthrax or anthrax spores, or a protein which is indicative of a particular disease state or of exposure to a particular disease, such as an immunoglobulin for instance an antibody.

Preferably, where the binding reagent binds the analyte, it is specific for the target analyte. However, it may be relatively non-specific, for example Protein A, where the target analyte is say an immunoglobulin such as IgG, provided that a binding moiety fused to the virus is specific for the target analyte.

Suitable specific binding reagents include antibodies or binding fragments thereof, as well as lectins. Antibodies may be monoclonal or polyclonal, but are preferably monoclonal.

The binding reagent is immobilised on the surface using conventional methods. For example Protein A may be used to bind antibodies or binding fragments which include the Fc region thereof.

The surface may be any convenient surface, such as the surface of a reaction plate or well, for instance an ELISA plate or well, in addition to beads such as magnetic beads, or membranes such as cellulose membranes which are used for example in dipstick assay tests, as are conventional in the art. Where appropriate, sites which are not occupied with binding reagent may be "blocked", for example with protein such as bovine serum albumin or milk protein, or with polyvinylalcohol or ethanolamine, or mixtures of these, as is conventional in the art.

In particular, the virus used in the method is a recombinant phage which expresses a binding moiety for the analyte or the binding reagent that is suitably a specific binding partner. In particular, the specific binding partner will comprise a single chain variable fragment of an antibody (scFV).

Recombinant phage expressing binding moieties may be' produced using conventional methods, as is well known in the production of phage libraries for phage displays as discussed above (see for example Antibody Engineering, R. Konterman & S. Dubel (eds) Springer Lab Manuals, Springer-Verlag, Berlin Heidelberg, 2001). However similar techniques may be used to produce other types of recombinant viruses.

Viruses such as phages may be added singly or as multi-specificity mixtures, where more than one analyte is being looked for. In the latter case, detection of multiple nucleic acid sequences, each characteristic of individual viruses is carried out subsequently.

The nucleic acid sequence of the virus is detected using a quantitative polymerase chain reaction (PCR). In this case, reagents, including primers, polymerases, nucleotides, and buffers as are well known, are added to the surface, and then subjected to thermal cycling as is conventional, in order to amplify any target nucleic acid sequence present.

The amplification product may then be detected using conventional methods such as gel electrophoresis, followed by visualisation using dyes.

Preferably the amplification reaction is carried out in such a way that the amplification product generates a detectable signal, and in particular a visible signal, for example a fluorescent signal, as it progresses. Many assay formats that produce such signals are known in the art. They may utilise reagents such as DNA binding agents such as intercalating dyes which emit radiation and particularly fluorescent radiation at greater intensity when they are intercalated into double stranded DNA, as well as probes and primers which include fluorescent labels, arranged to undergo fluorescent energy transfer (FET) and particularly fluorescent resonant energy transfer (FRET).

There are two commonly used types of FET or FRET probes, those using hydrolysis of nucleic acid probes to separate donor from acceptor, and those using hybridisation to alter the spatial relationship of donor and acceptor molecules.

Hydrolysis probes are commercially available as TaqMan^{™} probes. These consist of DNA oligonucleotides that are labelled with donor and acceptor molecules. The probes are designed to bind to a specific region on one strand of a PCR product. Following annealing of the PCR primer to this strand, *Taq* enzyme extends the DNA with 5' to 3' polymerase activity. *Taq* enzyme also exhibits 5' to 3' exonuclease activity. TaqMan^{™} probes are protected at the 3' end by phosphorylation to prevent them from priming *Taq* extension. If the TaqMan^{™} probe is hybridised to the product strand, an extending *Taq* molecule may also hydrolyse the probe, liberating the donor from acceptor as the basis of detection. The signal in this instance is cumulative, the concentration of free donor and acceptor molecules increasing with each cycle of the amplification reaction.

Hybridisation probes are available in a number of forms. Molecular beacons are oligonucleotides that have complementary 5' and 3' sequences such that they form hairpin loops. Terminal fluorescent labels are in close proximity for FRET to occur when the hairpin structure is formed. Following hybridisation of molecular beacons to a complementary sequence the fluorescent labels are separated, so FRET does not occur, and this forms the basis of detection.

Pairs of labelled oligonucleotides may also be used. These hybridise in close proximity on a PCR product strand-bringing donor and acceptor molecules together so that FRET can occur. Enhanced FRET is the basis of detection. Variants of this type include using a labelled amplification primer with a single adjacent probe.

Other methods for detecting amplification reactions as they occur are known however, and any of these may be used. Particular examples of such methods are described for example in WO 99/28500, British Patent No. 2,338,301, WO 99/28501 and WO 99/42611.

WO 99/28500 describes a very successful assay for detecting the presence of a target nucleic acid sequence in a sample. In this method, a DNA duplex binding agent and a probe specific for said target sequence, is added to the sample. The probe comprises a reactive molecule able to absorb fluorescence from or donate fluorescent energy to said DNA duplex binding agent. This mixture is then subjected to an amplification reaction in which target nucleic acid is amplified, and conditions are induced either during or after the amplification process in which the probe hybridises to the target sequence. Fluorescence from said sample is monitored.

An alternative form of this assay, which utilises a DNA duplex binding agent which can absorb fluorescent energy from the fluorescent label on the probe but which does not emit visible light, is described in co-pending British Patent Application No. 223563.8. Any of these assays may be used in the context of the assay method of the invention in order to detect the target nucleic acid sequence.

These assays are carried out in a quantitative manner as is well known in the art, for example by monitoring the signal from the amplification mixture at least once during each cycle of the amplification reaction. By carrying out the reaction in this way, the amount of virus present on the surface may be determined, and this may be related to the amount of analyte present in the original sample.

The particular sequence of the virus detected may be any characteristic sequence found therein. Where single specificity viruses are used in the assay, this may be any sequence found within the phage itself, as well as the sequence encoding the complementarity determining region (CDR) of the scFv, the "scaffolding" for the CDR of any recombinant virus, or other sequences introduced into the recombinant virus during its preparation such as antibiotic resistance genes.

If desired, specific marker sequences may be included in the virus in addition to those coding for the binding moiety. They may be introduced into the virus at the same time as the binding moiety, for example fused to the sequence encoding the binding moiety, or may be added in a separate transformation operation.

Where multi-specificity mixtures of viruses are used in the assay, then it is necessary to detect sequences which are characteristic of each particular virus, in order to determine whether specific analytes are present in the sample. In this case therefore, it is necessary to detect sequences such as the sequence encoding the scFv itself, or a specifically introduced marker sequence, as discussed above.

In this case, sequences common to viruses or recombinant viruses, such as phage DNA or RNA, or antibiotic resistance genes, may also be detected. Generally, there will always be some carry-over of viral nucleic acid, which can be used as internal reference sequences, ensuring that the PCR reaction has proceeded appropriately.

In this case multiplex PCR reactions using different signalling reagents or systems may be employed in order to detect the various sequences which are produced. This may be achieved, for example by labelling probes or primers used in the amplification reaction using different labels, for example, labels which fluoresce at different wavelengths. Examination of the signal from each label, for example at each of the different wavelength, is then carried out, if necessary with appropriate signal resolution where the wavelengths overlap.

Alternatively the assay is designed such that the amplicons produced by different PCR reactions hybridise to form duplexes or destabilise at different temperatures. Melting point analysis, for example using intercalating dyes that exhibit increased fluorescence when bound to double stranded DNA species, is a well-known technique. By adding such as dye to the reaction system, either during or after the assay process, and by monitoring fluorescence with a controlled change of temperature, the temperature at which the duplex structure of the amplicon breaks down or reforms can be determined, and this can be related to the presence of the particular amplicon and hence the particular virus which has bound.

The assay system of the invention thus provides a useful and reliable assay method.

Kits for use in the assay method described above form a further aspect of the invention.

In particular, the invention provides kit for detecting the presence of an analyte in a sample, said kit comprising solid body having immobilised on a surface thereof a binding reagent which either (a) binds said analyte, or (b) comprises said analyte or an analogue thereof; a virus which has been transformed so that it expresses a specific binding moiety for said binding reagent in competition to said analyte, and reagents capable of conducting a quantitative polymerase chain reaction for amplifying and detecting a nucleic acid of said virus as specified in the claims.

For instance, where the surface has immobilised thereon a binding reagent which binds said analyte, the virus suitably expresses and displays a binding partner which binds said binding reagent in competition to said analyte.

Alternatively, where the surface has immobilised thereon a binding reagent which comprises either the analyte or an analogue thereof, the virus is suitably one which expresses and displays a binding partner for said analyte.

Suitably, the solid body is a well in a plate, for instance a multi-well plate, but it may also be beads such as magnetic beads, or membranes, for example cellulose membranes as found in conventional dipstick type assays.

The kit may include more than one type of virus, in particular recombinant phages, for use in multi-specificity assays as discussed above.

Possible additional elements of the kit comprise reagents suitable for use in the detection of the nucleic acid sequences. In particular, the kit may comprise intercalating dyes, primers or probes for use the detection of the particular nucleic acid sequences as discussed above. For example, the kit may comprise primers which amplify sequences, which encode specific scFv sequences, or marker sequences which have been incorporated into the virus. In addition, or alternatively in the case of single specificity assays, the kits may include primers which are suitable for amplifying virus sequences, sequence which encode scaffolding of scFvs or antibiotic resistance genes which are present in recombinant virus.

The primers may suitably be labelled in such a way that the amplification product is directly detectable. For example, they may include fluorescent or other labels as described above.

The kit contains probes as defined in the claims.

Kits may also include intercalating dyes to assist with melting point analysis, where this is required in order to resolve multi-specificity assay results.

Recombinant viruses and in particular recombinant phages, which are transformed so that they express both a binding moiety and a marker sequence, are novel and as such form a further aspect of the invention.

### Description of the Drawings

Figure 1 illustrates diagrammatically a competitive assay including the invention;
Figure 2 is a graph of fluorescence -d(F1)/dt versus temperature when carrying out a PCR reaction of the TAQMAN® type;
Figure 3 is a graph of fluorescence (F1) against cycle number of series of samples at different dilutions using the assay of the invention; and
Figure 4 shows the results of an illustrative assay described hereinafter for *B*. *cereus* spores including a PCR for detecting phage DNA.

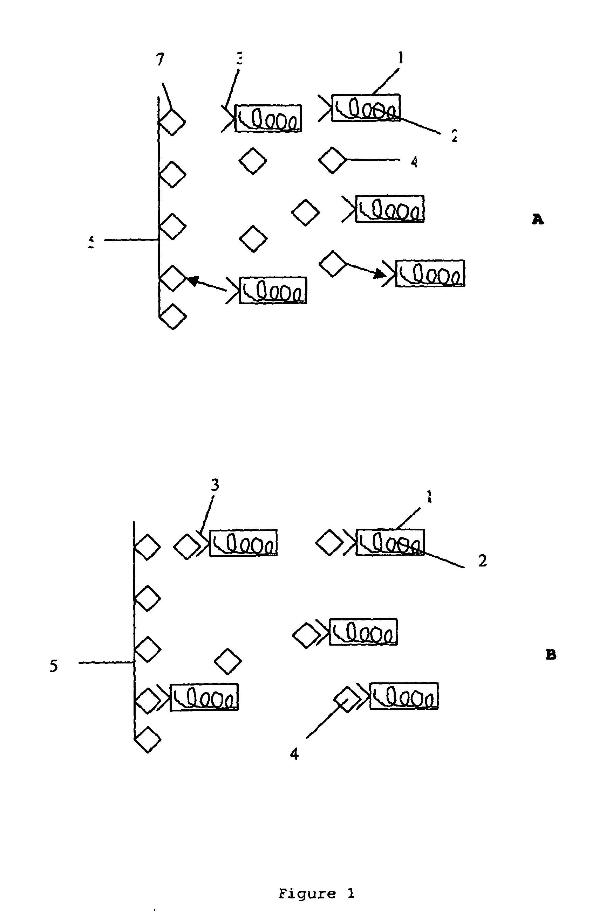

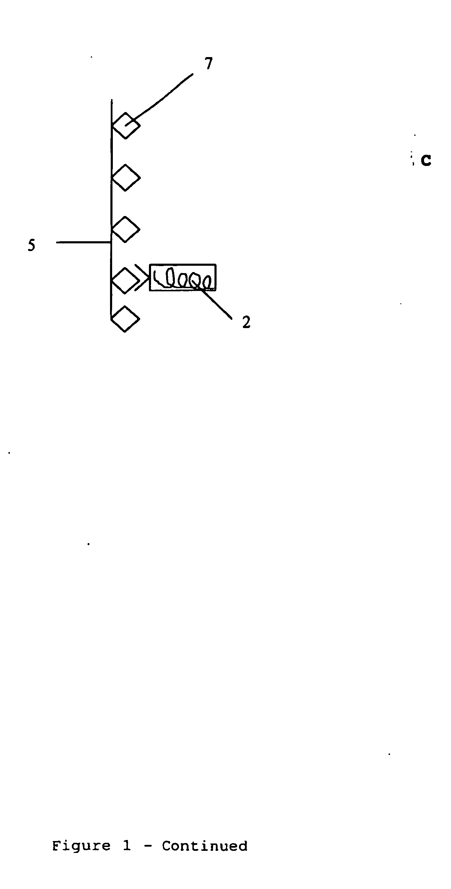

In the embodiment illustrated in Figure 1A, an analyte or an analogue thereof (7) capable of binding to the binding partner (3) of the phage (1) is immobilised on the surface (5). A sample under test to which the phage (1) has been added is incubated in the presence of this surface. Analyte (4) in the sample will bind to the binding partner (3) of the phage (1). Any phage which has undergone such binding is unable to bind to the immobilised analyte analogue (7) (B), and therefore will be washed away with the sample during a subsequent separation step. Detection of phage DNA (2) on the surface (5) following such a washing step will reveal lower levels than would otherwise be expected if no analyte were present.

Other competitive assay formats are possible as would be understood in the art.

### Illustration 1

### Demonstration of Assay using Bacillus cereus spores illustrating detection of virus nucleic acid using PCR

### 1) Plate format

A sample (50µl) comprising a suspension of *B.cereus* spores (1 x 10⁸ per ml) in distilled sterile water was added to each well of a blocked ELISA plate (Immulon microtitre ELISA plate) which was then placed in an oven at 37°C overnight to dry the spores onto the plates.

The plates were then washed three times with a wash solution comprising 0.05% v/v Tween 20 in phosphate buffered saline (PBS) or PBST.

A blocking buffer (200ml) comprising 2% w/v dry milk powder and 0.05% v/v Tween 20 PBS was added to each well. The plate was then sealed and incubated at room temperature for a minimum of 1 hour. It was then washed again three times in PBST.

A solution of primary antibody expressing M13 phage (1x 10⁹ transforming units per ml), wherein the primary antibody is a *B. cereus* specific single chain variable fragment (scFv), in PBST blocking buffer was prepared and at least 50µl added per well. PBST blocking buffer was added to one of the wells in place of the primary antibody expressing phage as a negative control.

The plate was incubated at 37°C for 1 hour, then washed 5 times in PBST. After drying, 50 µl dH₂O was added to each well. The plate was then boiled for 30 seconds to free the phage for PCR. After allowing the plate to cool briefly, and contents of the wells were transferred to a PCR tube, together with a conventional PCR mix (18 µl) including M13 phage specific primers and SybrGreen, used in accordance with the manufacturer's instructions.

The sample subjected to a series of thermal cycling steps on the Roche LightCycler as follows:
94°C for 0 seconds (melt);
62°C for 30 seconds (annealing phase);
72°C for 30 seconds (extension phase).

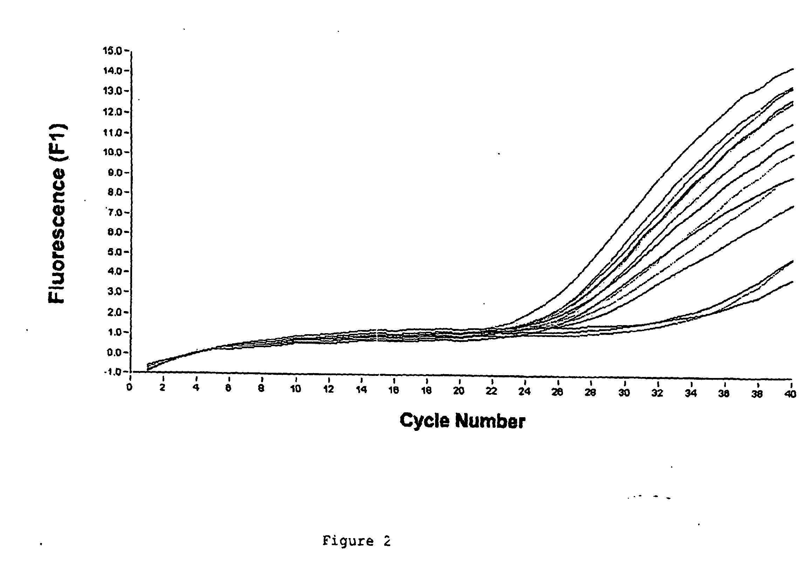

40 cycles were carried out. The fluorescent signal from the samples was monitored once per cycle at the end of the extension phase. The process was repeated with an increasingly dilute sample and the results are shown in Figure 2.

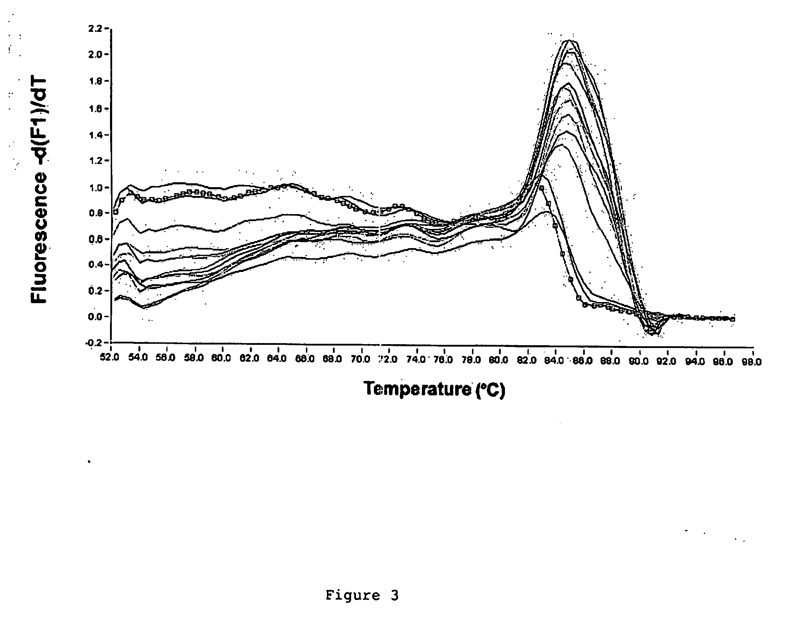

Negative control samples showed only a small increase in signal at the end of the cycling process. It was confirmed by a final melt curve analysis (Figure 3) that the signal from the negative control was due to non-specific products such as primer-dimers.

The results show however that the presence of bacterial spores in the samples was detectable using this method.

### Illustrative Example 2

### Detection of virus DNA on a surface

For use as a detection assay, a sample is added to a blocked-antibody coated ELISA plate and incubated at 37°C for 5 to 60 minutes.

Thereafter, the plate is washed with wash liquid from three to five times.

A suspension of filamentous phage expressing an scFv specific for the assay target is added to the plate, and incubated for 5-60 mins. After further washing (3-5 times), conventional PCR reagents are added, together with a suitable reporter system such as the SybrGreen dye mentioned above. However, other reporter mechanisms, for example using fluorescent reporter probes, such as TAQMAN® or other probes for in situ monitoring may be employed. The reaction mixture is thermally cycled to effect the amplification in the conventional way.

The PCR cycle number at which product appears (fluorescence threshold crossing point) is noted and correlated with the concentration of analyte in original sample.

It is possible to add more than one scFv with different specificities at the same time, to determine a range of targets. In such cases, melt profiles may be carried out to distinguish which one of the scFv is present and therefore has bound to the analyte. If desired, phage sequences or antibiotic resistance sequences found in the transformed phage may be used as an internal reference for the PCR.

### Illustrative Example 3

### Alternative Filtration Assay Format

In this embodiment, a liquid sample is passed through a 0.2 or 0.45 micron filter, depending upon the nature of the assay target, and the filter is then washed. Target analyte, for example bacterial spores, are retained on the filter. Subsequently, a suspension of filamentous phage expressing an scFv specific for the assay target is also passed through the filter, which is again washed. Any phage which binds to the target on the filter can then be detected, by PCR as described above.

### Illustrative Example 4

### Detection of phage displaying single chain antibodies directed against B cereus in an immunoassay format.

50µl of 10⁷ *B. cereus* spores/ml were diluted 10 fold in dH₂O down an Immulon 2 ELISA plate spores and dried onto the plate at 37°C overnight. This immobilised the spores on the plate so that they mirrored the situation in which an analyte was binding an immobilised antibody, as might occur in an assay for the spores.

The plate was then washed in dH₂O, three times. Each well was then blocked by the addition of 150µl of 1% blotto/phosphate buffered saline (PBS) and the plate was then incubated at 37 °C for 1 hour. The plate was washed in PBS-Tween, three times. 50µl of phage suspension in 1% blotto/PBS was added to each well and then the plate was incubated at 37°C for one hour, so that the phage bound to the spores on the plate. The plate was then washed in PBS-Tween 3 times, followed by three washes in dH₂O to remove unbound phage.

50µl of dH₂O was then added to each well and the plate was heated in boiling water for 30 seconds so as to elute the phage.

2µl from each well were then assayed by PCR using phage directed primers, amplify the lacI gene found within the M13 derived phage. Real-time PCR was performed using a Corbett RotorGene. Each tris-buffered reaction contained 0.5µM each of forward and reverse LacI primers, 0.3 µM of lacI specific TaqMan probe, 4 mM MgCl₂. Cycling parameters were 95° for 5 seconds and 60°C for 1 minute for 50 cycles. The primers, probe and target were as follows:

| | |
|---|---|
| *FORWARD PRIMER* | *5'-CGTGGTGGTGTCGA TGGTAG* |
| *REVERSE PRIMER* | *5'-TGTGCACCGCTTT* |
| *PROBE SEQUENCE* | *5*'- *ACGAAG CGGCGTCGAA GCCTG* |
| AMPLICON | 5'-CGTGGTGGTGTCGATGGTAGAACGA AGCGGC GTCGAAGCCTGTAAGCGGCGGTGCACA |

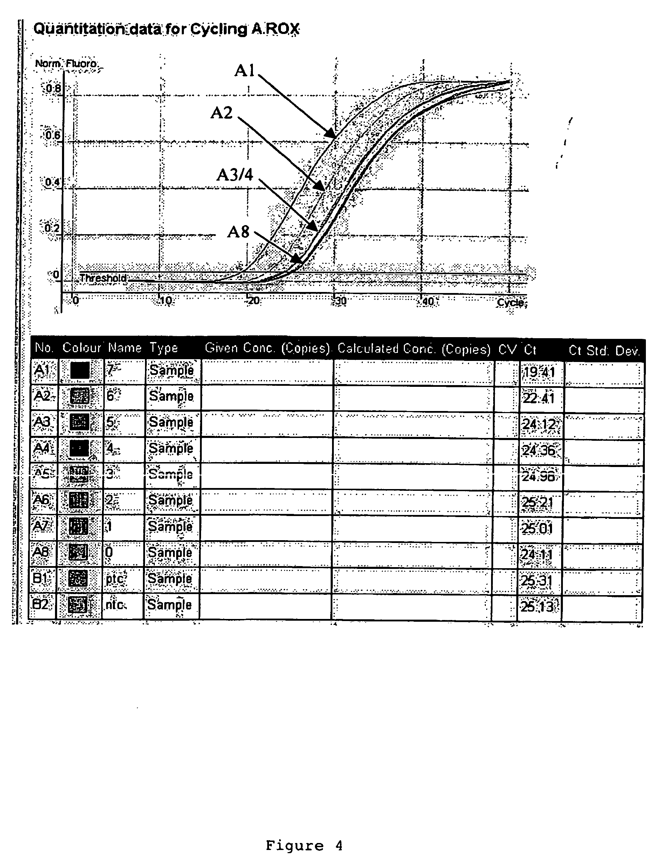

The results are shown in Figure 4. The results show that 10⁶ to 10³ spores per well were detectable above background level, even though the background level in this case was quite high. This was probably as a result of cross-contamination. There are shared genes between M13 derived phages and M13 derived cloning vectors used routinely in the lab. The sensitivity of the assay could be readily improved by setting up the phage PCR reaction in a lab that is free of M13 contamination or by choosing primers specific to phages displaying *B. cereus* antibodies.

## Claims

1. A method for determining the amount of an analyte in a sample, said method comprising contacting said sample with a surface having immobilised thereon a binding reagent which either (a) binds said analyte, or (b) comprises said analyte or an analogue thereof, and a virus which has been transformed so that it expresses and displays a binding moiety that binds said binding reagent in competition to said analyte, separating said surface from the sample, and carrying out a quantitative polymerase chain reaction to detect the amount of a nucleic acid sequence present within said virus on said surface, wherein at least one of the binding reagent or the binding moiety is specific for the analyte.

2. A method according to claim 1 where the immobilised binding reagent comprises the analyte or an analogue thereof, so that the binding moiety will bind either the analyte or the binding reagent, and the presence of analyte in the sample blocks the binding of the binding moiety to the binding reagent, so that a reduction in the amount of virus able to bind to the binding reagent is indicative of the presence of analyte in the sample.

3. A method according to claim 1 wherein the binding reagent binds the analyte, and also the binding moiety on the virus, so that the analyte and the binding moiety will compete for available sites on the surface, so that a reduction in the amount of virus able to bind to the binding reagent is indicative of the presence of analyte in the sample.

4. A method according to any one of the preceding claims wherein the surface is the surface of an ELISA plate or well, a magnetic bead or a membrane.

5. A method according to any one of the preceding claims wherein sites on the surface which are not occupied with binding reagent are blocked.

6. A method according to claim 1 wherein the analyte specific binding partner is a single chain variable fragment of an antibody (scFV) .

7. A method according to any one of the preceding claims wherein the virus comprises a multi-specificity mixture.

8. A method according to claim 7 wherein detection of multiple nucleic acid sequences, each characteristic of individual viruses is carried out.

9. A method according to claim 1 wherein the polymerase chain reaction is carried out in such a way that the amplification product generates a detectable signal as the reaction progresses, and the signal from the amplification mixture is monitored at least once during each cycle of the amplification reaction so as to determine the the amount of virus present on the surface.

10. A method according to claim 9 wherein the signal is a visible signal.

11. A method according to claim 9 or claim 10 wherein the polymerase chain reaction is carried out in the presence of a DNA binding reagent, or a primer or probe which is labelled with a fluorescent label.

12. A method according to claim 11 wherein the DNA binding reagent is an intercalating dye.

13. A method according to claim 11 wherein the reaction is carried out in the presence of a probe which comprises a DNA oligonucleotide that is labelled with a fluorescent energy donor and a fluorescent energy acceptor molecule, and wherein during the amplification, probe bound to target sequence is hydrolysed to liberate the donor and acceptor molecules.

14. A method according to any one of the preceding claims wherein virus nucleic acid is detected by amplifying a nucleic acid sequence which is characteristic of a particular virus used in the method.

15. A method according to claim 14 wherein the virus is a recombinant virus which has been transformed with a marker sequence, and this sequence is the sequence which is amplified.

16. A method according to claim 14 wherein sequences characteristic of more than one virus are detected.

17. A method according to any one of the preceding claims where more than one virus is used in the process, and a subsequent melting point analysis is conducted to determine which virus has bound during the assay.

18. A kit for determining the amount of an analyte in a sample, said kit comprising a solid body having immobilised on a surface thereof a binding reagent which either (a) binds said analyte, or (b) comprises said analyte or an analogue thereof; a virus which has been transformed so that it expresses a specific binding moiety for said binding reagent in competition to said analyte, and reagents capable of conducting a quantitative polymerase chain reaction for amplifying and detecting a nucleic acid of said virus wherein the reagents capable of conducting a quantitative polymerase chain reaction for amplifying and detecting a nucleic acid of said virus include a DNA probe which comprises a fluorescent energy donor molecule and a fluorescent energy acceptor molecule.

19. A kit according to claim 18 which comprises more than one type of transformed virus.

20. A kit according to claim 18 or claim 19 which comprises an intercalating dye.

## Patentansprüche

1. Verfahren zur Bestimmung der Menge eines Analyten in einer Probe, wobei das Verfahren umfasst das Inkontaktbringen der Probe mit einer Oberfläche, auf der ein Bindungsreagens immobilisiert ist, welches entweder (a) den Analyten bindet oder (b) den Analyten oder ein Analog davon umfasst, und einem Virus, das transformiert worden ist, so dass es eine Bindungskomponente exprimiert und displayed, die das Bindungsreagens in Konkurrenz zu dem Analyten bindet, Trennen der Oberfläche von der Probe und Durchführen einer quantitativen Polymerase-Kettenreaktion, um die Menge einer Nucleinsäuresequenz, die in dem Virus vorhanden ist, auf der Oberfläche zu bestimmen, wobei mindestens eines aus dem Bindungsreagens oder der Bindungskomponente für den Analyten spezifisch ist.

2. Verfahren nach Anspruch 1, worin das immobilisierte Bindungsreagens den Analyten oder ein Analog davon umfasst, so dass die Bindungskomponente entweder den Analyten oder das Bindungsreagens binden wird, und die Gegenwart von Analyt in der Probe die Bindung der Bindungskomponente an das Bindungsreagens blockiert, so dass eine Verringerung der Menge an Virus, das befähigt ist an das Bindungsreagens zu binden, ein Anzeichen für das Vorhandensein des Analyten in der Probe ist.

3. Verfahren nach Anspruch 1, worin das Bindungsreagens den Analyten bindet, und auch die Bindungskomponente auf dem Virus, so dass der Analyt und die Bindungskomponente um verfügbare Stellen an der Oberfläche konkurrieren, so dass eine Verringerung der Menge an Virus, das befähigt ist an das Bindungsreagens zu binden, ein Anzeichen für das Vorhandensein des Analyten in der Probe ist.

4. Verfahren nach irgendeinem der vorangehenden Ansprüche, worin die Oberfläche die Oberfläche einer ELISA-Platte, eines ELISA-Wells, eines magnetischen Teilchens oder einer Membran ist.

5. Verfahren nach irgendeinem der vorangehenden Ansprüche, worin die Stellen an der Oberfläche, die nicht von dem Bindungsreagens besetzt sind, blockiert sind.

6. Verfahren nach Anspruch 1, worin der Analyt-spezifische Bindungspartner ein einkettiges variables Fragment eines Antikörpers (scFV) ist.

7. Verfahren nach irgendeinem der vorangehenden Ansprüche, worin das Virus eine multi-spezifische Mischung umfasst.

8. Verfahren nach Anspruch 7, worin ein Nachweis auf multiple Nucleinsäuresequenzen, die jeweils charakteristisch für individuelle Viren sind, durchgeführt wird.

9. Verfahren nach Anspruch 1, worin die Polymerase-Kettenreaktion derart durchgeführt wird, dass das Amplifikationsprodukt mit fortschreitender Reaktion ein detektierbares Signal erzeugt und das Signal des Amplifikationsgemisches mindestens einmal während jedes Zyklus der Amplifikationsreaktion überwacht wird, um die Menge an Virus zu bestimmen, die auf der Oberfläche vorhanden ist.

10. Verfahren nach Anspruch 9, worin das Signal ein sichtbares Signal ist.

11. Verfahren nach Anspruch 9 oder Anspruch 10, worin die Polymerase-Kettenreaktion in Gegenwart eines DNA-Bindungsreagens oder eines Primers oder einer Sonde durchgeführt wird, die mit einem Fluoreszenzmarker markiert ist.

12. Verfahren nach Anspruch 11, worin das DNA-Bindungsreagens ein interkalierender Farbstoff ist.

13. Verfahren nach Anspruch 11, worin die Reaktion in Gegenwart einer Sonde durchgeführt wird, die ein DNA-Oligonucleotid umfasst, das mit einem Fluoreszenzenergie-Donor- und einem Fluoreszenzenergie-Akzeptormolekül markiert ist, und worin während der Amplifikation die an die Targetsequenz gebundene Sonde hydrolysiert wird, um die Donor- und Akzeptormoleküle freizusetzen.

14. Verfahren nach irgendeinem der vorangehenden Ansprüche, worin Virus-Nucleinsäure detektiert wird durch Amplifizieren einer Nucleinsäuresequenz, die für ein bestimmtes, in dem Verfahren verwendetes Virus charakteristisch ist.

15. Verfahren nach Anspruch 14, worin das Virus ein rekombinantes Virus ist, das mit einer Markersequenz transformiert worden ist, wobei diese Sequenz die Sequenz ist, die amplifiziert wird.

16. Verfahren nach Anspruch 14, worin Sequenzen, die für mehr als ein Virus charakteristisch sind, detektiert werden.

17. Verfahren nach irgendeinem der vorangehenden Ansprüche, worin mehr als ein Virus in dem Verfahren verwendet werden, und eine anschließende Schmelzpunktanalyse durchgeführt wird, um festzustellen, welches Virus während des Assay gebunden hat.

18. Kit zur Bestimmung der Menge eines Analyten in einer Probe, wobei das Kit umfasst einen festen Körper, der auf einer Oberfläche ein Bindungsreagens immobilisiert aufweist, welches entweder (a) den Analyten bindet oder (b) den Analyten oder ein Analog davon umfasst; ein Virus, das transformiert worden ist, so dass es eine spezifische Bindungskomponente für das Bindungsreagens in Konkurrenz zu dem Analyten exprimiert, und Reagentien zur Durchführung einer quantitativen Polymerase-Kettenreaktion zum Amplifizieren und Detektieren einer Nucleinsäure des Virus, wobei die zum Durchführen einer quantitativen Polymerase-Kettenreaktion befähigten Reagentien zum Amplifizieren und Detektieren einer Nucleinsäure des Virus eine DNA-Sonde umfassen, welche ein Fluoreszenzenergie-Donormolekül und ein Fluoreszenzenergie-Akzeptormolekül umfasst.

19. Kit nach Anspruch 18, welches mehr als eine Art von transformiertem Virus umfasst.

20. Kit nach Anspruch 18 oder Anspruch 19, welches einen interkalierenden Farbstoff umfasst.

## Revendications

1. Procédé permettant de déterminer la quantité d'un analyte dans un échantillon, ledit procédé comprenant la mise en contact dudit échantillon avec une surface sur laquelle est immobilisé un réactif de liaison qui soit (a) se lie audit analyte, soit (b) comprend ledit analyte ou un analogue de celui-ci, et un virus qui a été transformé de manière à exprimer et à présenter un fragment de liaison qui se lie audit réactif de liaison de manière compétitive avec ledit analyte, la séparation de ladite surface de l'échantillon et la mise en oeuvre d'une amplification en chaîne par polymérase quantitative pour détecter la quantité d'une séquence d'acides nucléiques présente dans ledit virus sur ladite surface, dans lequel au moins un parmi le réactif de liaison où le fragment de liaison est spécifique de l'analyte.

2. Procédé selon la revendication 1, où le réactif de liaison immobilisé comprend l'analyte ou un analogue de celui-ci, de sorte que le fragment de liaison se liera soit à l'analyte, soit au réactif de liaison, et la présence d'un analyte dans l'échantillon bloque la liaison du fragment de liaison sur le réactif de liaison, de sorte qu'une réduction de la quantité de virus capable de se lier au réactif de liaison indique la présence d'un analyte dans l'échantillon.

3. Procédé selon la revendication 1, dans lequel le réactif de liaison se lie à l'analyte, de même que le fragment de liaison sur le virus, de sorte que l'analyte et le fragment de liaison seront en compétition pour les sites disponibles se trouvant sur la surface, de sorte qu'une réduction de la quantité de virus capable de se lier au réactif de liaison indique la présence d'un analyte dans l'échantillon.

4. Procédé selon l'une quelconque des revendications précédentes, dans lequel la surface est la surface d'un puits ou d'une plaque ELISA, d'une bille magnétique ou d'une membrane.

5. Procédé selon l'une quelconque des revendications précédentes, dans lequel les sites se trouvant sur la surface qui ne sont pas occupés par le réactif de liaison sont bloqués.

6. Procédé selon la revendication 1, dans lequel le partenaire de liaison spécifique de l'analyte est un fragment variable à chaîne simple d'un anticorps (scFV).

7. Procédé selon l'une quelconque des revendications précédentes, dans lequel le virus comprend un mélange de spécificités multiples.

8. Procédé selon la revendication 7, dans lequel une détection de multiples séquences d'acides nucléiques, chacune étant caractéristique de virus individuel, est réalisée.

9. Procédé selon la revendication 1, dans lequel l'amplification en chaîne par polymérase est menée d'une telle manière que le produit d'amplification génère un signal détectable quand la réaction évolue, et le signal provenant du mélange d'amplification est contrôlé au moins une fois pendant chaque cycle de la réaction d'amplification afin de déterminer la quantité de virus présent sur la surface.

10. Procédé selon la revendication 9, dans lequel le signal est un signal visible.

11. Procédé selon la revendication 9 ou la revendication 10, dans lequel l'amplification en chaîne par polymérase est menée en présence d'un réactif se liant à l'ADN, ou d'une amorce ou d'une sonde qui est marquée avec un marqueur fluorescent.

12. Procédé selon la revendication 11, dans lequel le réactif se liant à l'ADN est un colorant d'intercalation.

13. Procédé selon la revendication 11, dans lequel la réaction est menée en présence d'une sonde comprenant un oligonucléotide d'ADN marqué avec une molécule fluorescente de donneur d'énergie et une molécule fluorescente d'accepteur d'énergie, et dans lequel pendant l'amplification, la sonde liée à la séquence cible est hydrolysée pour libérer les molécules de donneur et d'accepteur.

14. Procédé selon l'une quelconque des revendications précédentes, dans lequel l'acide nucléique du virus est détecté en amplifiant une séquence d'acides nucléiques qui est caractéristique d'un virus particulier utilisé dans le procédé.

15. Procédé selon la revendication 14, dans lequel le virus est un virus recombinant qui a été transformé avec une séquence de marqueur, et cette séquence est la séquence qui est amplifiée.

16. Procédé selon la revendication 14, dans lequel des séquences caractéristiques de plus d'un virus sont détectées.

17. Procédé selon l'une quelconque des revendications précédentes, où plus d'un virus est utilisé dans le procédé, et une analyse ultérieure du point de fusion est menée pour déterminer quel virus s'est lié pendant l'essai.

18. Kit permettant de déterminer la quantité d'un analyte dans un échantillon, ledit kit comprenant un corps solide possédant une surface sur laquelle est immobilisé un réactif de liaison qui soit (a) se lie audit analyte, soit (b) comprend ledit analyte ou un analogue de celui-ci ; un virus qui a été transformé de manière à exprimer un fragment de liaison spécifique pour ledit réactif de liaison en compétition avec ledit analyte, et des réactifs capables de mener une amplification en chaîne par polymérase quantitative pour amplifier et détecter un acide nucléique dudit virus, dans lequel les réactifs capables de mener une amplification en chaîne par polymérase quantitative pour amplifier et détecter un acide nucléique dudit virus comprennent une sonde à ADN comprenant une molécule fluorescente de donneur d'énergie et une molécule fluorescente d'accepteur d'énergie.

19. Kit selon la revendication 18, comprenant plus d'un type de virus transformé.

20. Kit selon la revendication 18 ou la revendication 19, comprenant un colorant d'intercalation.
